# EUROPÄISCHE PATENTSCHRIFT

(11) **EP 1 554 383 B1**
(45) Veröffentlichungstag und Bekanntmachung des Hinweises auf die Patenterteilung: **20.01.2010**
(21) Anmeldenummer: 03769329.8
(22) Anmeldetag: 26.09.2003
(51) Int. Cl.: C12N 15/30, C12N 15/863, A61K 39/002

(54) **REKOMBINANTE MVA-STAMME ALS POTENTIELLE IMPFSTOFFE GEGEN P. FALCIPARUM -MALARIA**
RECOMBINANT MVA STRAINS AS POTENTIAL VACCINES AGAINST P. FALCIPARUM MALARIA
SOUCHES DE MVA DE RECOMBINAISON SERVANT DE VACCINS POTENTIELS CONTRE LE PALUDISME A P.FALCIPARUM

(30) Priorität: 23.10.2002 DE 10249390
(43) Veröffentlichungstag der Anmeldung: 20.07.2005
(73) Patentinhaber: Ruprecht-Karls-Universität Heidelberg, 69117 Heidelberg (DE); Helmholtz Zentrum München Deutsches Forschungszentrum für Gesundheit und Umwelt (GmbH), 85764 Neuherberg (DE)
(72) Erfinder: SUTTER, Gerd, 80803 München (DE); BUJARD, Hermann, 69120 Heidelberg (DE); WESTERFELD, Nicole, 3012 Bern (CH); MIAO, Jun, 69118 Heidelberg (DE)
(74) Vertreter: Grünecker, Kinkeldey, Stockmair & Schwanhäusser Anwaltssozietät
(86) Internationale Anmeldenummer: PCT/EP2003/010723
(87) Internationale Veröffentlichungsnummer: WO 2004/038024

(56) Entgegenhaltungen:
- WO-A-02/24224
- DE-A- 19 640 817
- US-B1- 6 214 353
- SHUTONG Y ET AL: "Addition of the MSA1 signal and anchor sequences to the malaria merozoite surface antigen 1 C-terminal region enhances immunogenicity when expressed by recombinant vaccinia virus" VACCINE, BUTTERWORTH SCIENTIFIC. GUILDFORD, GB, Bd. 15, Nr. 12-13, 1. August 1997 (1997-08-01), Seiten 1303-1313, XP004089472 ISSN: 0264-410X
- SCHNEIDER J ET AL: "ENHANCED IMMUNOGENICITY FOR CD8+ T CELL INDUCTION AND COMPLETE PROTECTIVE EFFICACY OF MALARIA DNA VACCINATION BY BOOSTING WITH MODIEFIED VACCINIA VIRUS ANKARA" NATURE MEDICINE, NATURE PUBLISHING, CO, US, Bd. 4, Nr. 4, April 1998 (1998-04), Seiten 397-402, XP000739989 ISSN: 1078-8956
- OBER B T ET AL: "Immunogenicity and safety of defective vaccinia virus Lister: Comparison with modified vaccinia virus Ankara" JOURNAL OF VIROLOGY, Bd. 76, Nr. 15, August 2002 (2002-08), Seiten 7713-7723, XP002270126 ISSN: 0022-538X
- GOOD M F ET AL: "Immune effector mechanisms in malaria" CURRENT OPINION IN IMMUNOLOGY, CURRENT BIOLOGY LTD, XX, Bd. 11, Nr. 4, August 1999 (1999-08), Seiten 412-419, XP004257559 ISSN: 0952-7915
- RAMSHAW I A ET AL: "The prime-boost strategy: exciting prospects for improved vaccination" IMMUNOLOGY TODAY, ELSEVIER PUBLICATIONS, CAMBRIDGE, GB, Bd. 21, Nr. 4, April 2000 (2000-04), Seiten 163-165, XP004194963 ISSN: 0167-5699
- WOEHLBIER U. ET AL: 'Analysis of antibodies directed against merozoite surface protein 1 of the human malaria parasite Plasmodium falciparum.' INFECTION AND IMMUNITY Bd. 74, Nr. 2, Februar 2006, Seiten 1313 - 1322, XP002432920
- KUTINOVA L. ET AL: 'Influence of the parental virus strain on the virulence and immunogenicity of recombinant vaccinia viruses expressing HBV preS2-S protein or VZV glycoprotein I.' VACCINE Bd. 14, Nr. 11, August 1996, Seiten 1045 - 1052, XP004057639
- KUTINOVA L. ET AL: 'Effect of virulence on immunogenicity of single and double vaccinia virus recombinants expressing differently immunogenic antigens: Antibody-response inhibition induced by immunization with a mixture of recombinants differing in virulence' J GENERAL VIROLOGY Bd. 80, Nr. 11, November 1999, Seiten 2901 - 2908
- SUTTER G. ET AL: 'Stable expression of the vaccinia virus K1L gene in rabbit cells complements the host range defect of a vaccinia virus mutant.' J VIROLOGY Bd. 68, Nr. 7, 1994, Seiten 4109 - 4116
- SUTTER G. ET AL: 'Vaccinia Virus MVA for delivery of recombinant genes: evaluation in experimental tumar therapy.' INFECTION, MMV MEDIZIN VERLAG Bd. 28, November 2000, MUENCHEN, DE, Seite 12, XP008074482
- SUTTER G.; SCHWAB C.: 'Vaccinia vectors as candidate vaccines: the delopment of modified vaccinia virus ankara for antigen delivery.' CURRENT DRUG TARGETS. INFECTIOUS DISORDERS Bd. 3, Nr. 3, September 2003, Seiten 262 - 271
- SCHNEIDER J. ET AL: 'A prime-boost immunisation regimen using DNA followed by recombinant modified vaccinia virus Ankara induces strong cellular immune responses against the Plasmodium falciparum TRAP antigen in chaimpanzees.' VACCINE Bd. 19, Nr. 32, 14 September 2001, Seiten 4595 - 4602, XP004303150
- SIDDIQUI W.A. ET AL: 'Merozoite surface coat precursor protein completely protects Aotus monkeys against Plasmodium falciparum malaria.' PNAS Bd. 84, 01 Mai 1987, Seiten 3014 - 3018, XP002057621

## Beschreibung

### Einleitung

Die Herstellung rekombinanter *Vaccinia-Viren* des Stamms MVA *(Modifiziertes Vaccinia Virus Ankara)* zur rekombinanten Herstellung des vollständigen Malaria-Antigens gp190/MSP-1 des *Malariaerregers Plasmodium falciparum* sowie einzelner natürlich vorkommender Domänen und Teile derselben werden hier beschrieben. Ferner betrifft die Erfindung die Nutzung der rekombinanten MVA, welche die synthetische DNA-Sequenz des Malaria-Antigens sowie Teile derselben integriert in das Virus-Genom tragen als Impfstoff zur Immunisierung gegen Malaria.

Malaria zählt zu den gefährlichsten Infektionskrankheiten der Welt. Schätzungen der Weltgesundheitsorganisation (WHO) zufolge werden jährlich 400 bis 900 Millionen Krankheitsepisoden registriert. Nach Informationen der Multilateralen Initiative gegen Malaria (MIM) sterben zwischen 700.000 und 2,7 Millionen Menschen jedes Jahr an der Infektion (MIM, 2001). Dabei sind in 99 Ländern 40% der Weltbevölkerung einem Malariarisiko ausgesetzt. Verursacht wird die Krankheit durch einzellige Protozoen der Gattung *Plasmodium* aus dem Phylum *Apicomplexa.* Es gibt vier Arten, die den Menschen infizieren: *Plasmodium malariae,* verantwortlich für Malaria quartana, *Plasmodium vivax* und *Plasmodium ovale,* beide verursachen Malaria tertiana und schließlich *Plasmodium falciparum,* der Erreger der Malaria tropica und letztlich verantwortlich für fast alle tödlichen Infektionen.
Ihre Verbreitung nimmt derzeit wieder zu. Dies ist vor allem auf eine intensive Resistenzbildung der Malaria-Erreger zurückzuführen, die dadurch gefördert wird, dass die zur Therapie benutzen Medikamente ebenfalls zur Prophylaxe empfohlen und eingesetzt werden. Neben der Suche nach neuen Chemotherapeutika konzentriert sich die Forschung auf die Entwicklung von Impfstoffen, da im Laufe von Malaria-Infektionen im Menschen Immunitätsmechanismen einsetzen, die sich in einer erhöhten Widerstandsfähigkeit gegenüber den Plasmodien äußert, wie sich in der Entwicklung verschiedener Arten von Immunität bei Menschen in Malaria-epidemischen Regionen zeigt

### MSP-1 als potentieller Impfstoff

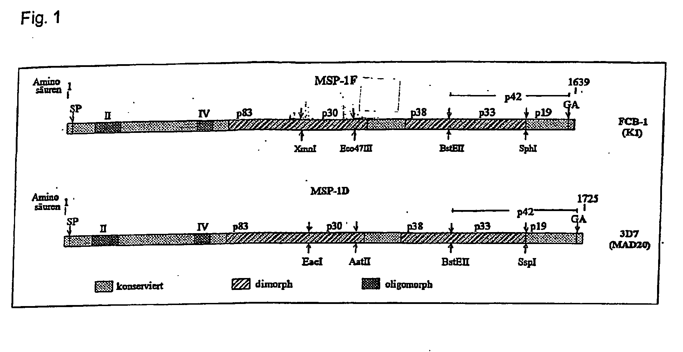

MSP-1, das Hauptoberflächenprotein von Merozoiten, der invasiven Form der Blutstadien der Malaria-Erreger, ist ein 190 - 220 kDa Protein. Dieses Protein wird spät während der Entwicklung der Merozoiten in kleinere Proteinfragmente prozessiert, die bis zur Invasion von Erythrozyten durch die Parasiten als Komplex verankert über einen Glycosylphosphatidyl-Inositol-Anker auf der Merozoiten-Obertläche vorliegen und isoliert werden können.
Die Sequenzen der MSP-1-Proteine verschiedener *P. falciparum-Stämme* fallen in zwei Gruppen, die nach zwei repräsentativen Isolaten K1 und MAD20 benannt wurden. Insgesamt besteht das Protein aus mehreren hochkonservierten Regionen, aus dimorphen Bereichen, die sich jeweils einer der beiden zuordnen lassen und aus zwei relativ kleinen oligomorphen Blöcken im N-terminalen Teil des Proteins (Fig. 1; Tanabe et al., 1987).

Die Immunisierung von Mäusen mit dem aus *P.* yoelii-Parasiten aufgereinigten Protein schütze die Tiere vor der andernfalls tödlichen Infektion (Holder and Freeman, 1981). Auch der Transfer von monoklonalen Antikörpern gegen MSP-1 von *P. yoelii* vermittelte Schutz im Mausmodell (Majarian et al., 1984).
Neben Studien an Mäusen wurden auch *Saimiri* und Aotus-Affen mit nativem, immunaffinitätsgereinigtem MSP-1 immunisiert. In diesen Versuchen schützte das aus *P. falciparum* gewonnene Protein partiell (Perrin et al., 1984) bzw. völlig (Siddiqui et al., 1987) gegen die folgende Infektion mit dem Parasiten.
Eine Aufreinigung von nativem Material aus *Plasmodien* ist allerdings aufwendig und lässt keine Produktion im großen Maßstab zu. Daher konzentriert sich die Impfstoff-Forschung auf die Entwicklung rekombinanter Vakzine.
So wurden Mäuse erfolgreich mit aus *E*. *coli* oder *Saccharomyces cerevisiae* gereinigtem MSP-1-19 immunisiert (Daly and Long, 1993; Ling et al., 1994; Tian et al., 1996; Hirunpetcharat et al., 1997), ebenso wie MSP-1-19 tragendes *Mycobacterium bovis* (Matsumoto et al., 1999). Alternativ zur Immunisierung mit nativen oder rekombinanten Proteinen wurde auch für MSP-1-19-kodierende DNA als Impfstoff eingesetzt und schütze immunisierte Mäuse vor der Infektion mit *P. chabaudi* (Wunderlich et al., 2000).
Die Immunisierungen von Affen mit rekombinantem MSP-1-19 und MSP-1-42 aus *P. falciparum* vermittelte teilweise Schutz (Kumar et al., 1995; Egan et al., 2000; Chang et al., 1996; Stowers et al., 2001). Die Interpretation von Immunsierungsversuchen an Affen ist allerdings nur bedingt möglich, da eine statistische Auswertung der Ergebnisse auf Grund der geringen Versuchstierzahlen nicht gegeben ist.
In Phase I und II Studien zu MSP-1-Fragmenten als Impfstoff wurde deren Immunogenität auch im Menschen gezeigt. Dabei handelte es sich um p19 aus *P. falciparum* fusioniert an ein T-Helfer-Zell-Epitope des Tetanustoxins (Keitel et al., 1999) und die MSP-1-Blöcke 3 und 4 (Saul et al., 1999; Genton et al., 2000).
Einige epidemiologische Studien in endemischen Gebieten zeigten bei Erwachsenen eine Korrelation zwischen Antikörpertitem gegen MSP-1 und der Immunität gegen Malaria (Tolle et al., 1993; Riley et al., 1992; Riley et al., 1993).
Diese Untersuchungen zusammen mit den Immunisierungsstudien an Tieren belegen, dass es sich bei MSP-1 um einen vielversprechenden Kandidaten zur Entwicklung eines Malaria-Impfstoffs handelt.
Generell lassen sich diese Studien in zwei Ansätze unterscheiden, entweder wurde aus Parasiten aufgereinigtes Material oder in heterologen Systemen gewonnenes Material eingesetzt.
Sowohl für Funktionsuntersuchungen als auch für den Einsatz als Impfstoff müssen Proteine in guter Ausbeute, hoher Qualität und reproduzierbar herzustellen sein. Zwar läßt sich MSP-1 aus Parasiten aufreinigen, allerdings ist dies nur im kleinen Maßstab unter großem Aufwand möglich und daher für die Gewinnung von MSP-1 nach den genannten Kriterien auf diesem Weg nicht durchführbar.

*Vaccinia*-Viren gehören zur Gattung *Orthopoxvirus* in der Unterfamilie der *Chordopoxvirinae.* Es handelt sich bei den Pocken-Viren um komplexe Viren, die mit einem doppelsträngigen DNA-Genom von ca. 200 kb und einer Größe von 250 x 350 nm zu den größten bekannten Viren gehören. Sie bestehen aus einem quaderförmigen Virion, das von einer Membranhülle umgeben ist. In der Wirtszelle verläuft die Replikation und Generation der Pocken-Viren ausschließlich im Zytoplasma (zur Übersicht Moss et al., 1996). Dabei besitzen *Vaccinia*-Viren ein sehr breites Wirtszellspektrum, sie infizieren nahezu alle Zellen sowohl von Menschen als auch Tieren stammend. 1953 isolierte und reinigte Anton Mayr den Dermovaccinia-Stamm Chorioallantois Vaccinia Ankara (CVA). Dieses Virus wurde über fortlaufende Passage auf Hühner-Embryo-Fibroblasten weiter vermehrt, und heraus kam ein attenuiertes Virus, das in Tier und Mensch keine Virulenz mehr zeigte (Stickl et al., 1974). Ungeachtet dessen konnte das Virus weiterhin zur Immunprophylaxe gegen durch Orthopockenviren hervorgerufenen Erkrankungen bei Mensch und Tier eingesetzt werden (Stickl et al., 1974). Nach seinem Ursprungsort wurde dieses Virus *Modifiziertes Vaccinia-Virus Ankara* (MVA) genannt.
Molekufargenetisch betrachtet hat das Virus während der über 570 Passagen auf Hühner-Embryo-Fibroblasten 31 kb DNA-Sequenz seines Genoms, hauptsächlich in Form sechs großer Deletionen verloren, darunter wenigstens zwei Gene, die das Wirtszellspektrum und damit die Replikationsfähigkeit des Virus bestimmen (Meyer et al., 1991). Die Bildung infektiöser Viruspartikel ist bei der MVA-Infektion in den meisten von Säugetieren stammenden Zellen, einschließlich Zellen von Menschen, erst sehr spät im Infektionszyklus auf der Stufe des Virionenzusammenbaus blockiert, d.h. virale Gene unter der Kontrolle von Promotoren sowohl zur frühen als auch intermediären und späten Transkription können auch in nicht permissiven Zellen exprimiert werden. Das unterscheidet MVA von anderen attenuierten und replikationsdefizienten Pockenviren, wie z. B. Vaccinia-Virus NYVAC oder Kanarienpockenvirus ALVAC, deren Infektion in den meisten vom Säugetier stammenden Zellen bereits vor der viralen DNA-Replikation unterbrochen ist (Tartaglia et al., 1992, Sutter and Moss, 1992).

Zur Malariaimpfstoffentwicklung wurden verschiedene rekombinante Vaccinia-Viren eingesetzt, dabei wurden replikationskompetente Viren des Typs Western Reserve und Copenhagen und attenuierte Viren der Typen NYVAC, ALVAC oder COPAC verwendet (Kaslow et al., 1991; Etlinger and Altenburger, 1991; Aidoo et al., 1997; Allsopp et al., 1996).
Im Zusammenhang mit dem attenuierten Vaccinia-Virus MVA wurden lediglich rekombinante Viren beschrieben, welche CSP aus dem Nager-Parasiten *Plasmodium berghei* als Malaria-Antigen tragen (Schneider et al., 1998; Plebanski et al., 1998; Degano et al., 1999; Gilbert et al., 1999).

Ferner sind rekombinante Vaccinia-Viren beschrieben, die eine MSP-1-kodierende Sequenz enthalten. Die Autoren einer Publikation geben an, die native MSP-1-kodierende Sequenz in das Genom des Virustyps Western Reserve integriert zu haben, unterstützen diese Aussage aber nicht experimentell (keine Restriktionsanalysen, PCR, Nothemblot-, Westemblotanalysen etc.). Eine Immunisierung von Saimiri-Affen mit diesen rekombinanten Viren führte nicht zur Bildung MSP-1-spezifischer Antikörper und blieb überdies auch ohne messbaren Einfluss auf die humorale Immunantwort gegen MSP-1 nach einer *P. falciparum*-Infektion (Pye et al., 1991).
In einer weiteren Veröffentlichung wird der Vektor NYVAC-Pf7 beschrieben, der unter anderem *msp-1* aus *P. falciparum* exprimiert. Die Seren von zwei der sechs immunisierten Rhesusaffen zeigen in der Westernblotanalyse keine In der Publikation erkennbaren Signale gegen natives MSP-1. Die Signale von drei weiteren Tieren erkennen lediglich Teile des Proteinkompexes, und nur das Serum eines der immunisierten Tiere erkennt ein breiteres Bandenspektrum. Insgesamt erscheinen jedoch auch diese Signale schwach. Quantitative Analysen zu MSP-1-spezifischen Antikörpern durch ELISA wurden nicht angeführt (Tine et al., 1996).
In Humanversuchen der Phase I/IIa mit NYVAC-Pf7 wurden weder eine zelluläre Immunantwort gegen MSP-1 noch eine durch ELISA nachweisbare humorale Immunantwort nachgewiesen (Ockenhouse et al., 1998).
Im Gegensatz dazu weisen Tine et al. (1996) intaktes MSP-1 in der Westemblot-Analyse nach, wobei widersprüchlich erscheint, dass MSP-1 mittels *P. falciparum* Signalsequenzen (vgl. Publikationen von Moran and Caras, 1994, Yang et al., 1997) aus der Zelle transportiert wird.

Der vorliegenden Erfindung liegt daher die Aufgabe zu Grunde ein rekombinantes Vaccinia-Virus bereitzustellen, das fähig ist
- DNA-Sequenzen, welche MSP-1 aus *P, falciparum* bzw. Teibereiche desselben kodieren, stabil Integriert zu enthalten,
- diese Sequenzen effizient und reproduzierbar zu exprimieren und damit
- MSP-1 Protein in sekretierter oder Oberflächen-verankerter Form herzustellen, um
- einen Wirt zu Immunisieren und dabei
- eine zelluläre und humorale Immantwort hervorzurufen.

Die der vorliegenden Erfindung zu Grunde liegende Aufgabe wird gelöst durch die Bereitstellung eines Vakzins, umfassend
a) ein rekombinantes MVA-Virus, umfassend eine ein Fragment oder Fragmente von *Plasmodium falciparum* MSP-1 Protein kodierende Nukleinsäure; und
b) einen pharmakologischen verträglichen Träger,
wobei das durch die Nukleinsäure kodierte MSP-1 Fragment bzw. die MSP-1 Fragmente ausschließlich ausgewählt ist/sind aus:
- p42;
- p42 und p38;
- p83, p30, p42 und p38.

Erfindungsgemäß bedeutet der Ausdruck "Virus auf MVA-Basis" ein von MVA abgeleitetes Virus, das in nicht-essentiellen Regionen des Virusgenoms eine oder mehrere Mutationen aufweist Die essentiellen Regionen des MVA-Virus sind hierbei alle Genomabschnitte des MVA-Virus, die für den Erhalt der viralen Genexpression und die Vermehrungsfähigkeit des MVA-Virus notwendig sind, hierzu zählen z. b. die für virale RNA-Polymerase-Untereinheiten bzw. die virale DNA-Polymerase kodierenden Gensequenzen. Vorzugsweise ist das Virus auf MVA-Basis das MVA-Virus.

Das aus dem Stand der Technik bekannte *Vaccinia*-System NYVAC-Pf7 beruht auf dem Basisvirus NYVAC, welches ursprünglich dem *Vaccinia*-Virus-Stamm Copenhagen entstammt und durch die gezielte Deletion von 18 offenen Leserastem attenuiert wurde. Jedoch ist bei NYVAC in Säugerzellen die DNA-Replikation blockiert (Tartaglla et al., 1992), während bei MVA die Virusassemblierung unterbunden wird. Dies hat den Vorteil, dass im Gegensatz zu NYVAC In MVA die späte Genexpression stattfindet, welche für die Expression rekombinanter Gene genützt werden kann. MVA kann daher sowohl während der frühen Transkriptionsphase bevorzugt eine zytotoxische T-Zell-Antwort induzierten als auch den humoralen Zweig der Immunantwort durch die hohe Protein-Expression während der späten Phase stimulieren.

MVA, das bereits während der Pockenschutz-Impfkampagne umfangreich eingesetzt wurde, gilt als sehr sicheres Impfvirus am Menschen (Stickl et al., 1974).

Effindungsgemäß wird ein rekombinantes MVA-Virus, umfassend wenigstens eine ein Fragment oder Fragmente von *P. falciparum* MSP-1 Protein kodierende Nukleinsäure.

Die MSP-1-Aminosäuresequenz ist aus öffentlich zugänglichen Datenbanken erhältlich. 3D7 (MAD20-Isolat): CAA84556; FCB-1 (K1-Isolat): CAB36903. Beides aus der NIH-Datenbank (http://www.ncbi.nlm.nih.gov).

Besonders bevorzugt ist die MSP-1-Fragment oder MSP-1 Fragmente kodierende Nukleinsäure eine in ihrem AT-Gehalt verringerte Nukleinsäure wie beschrieben in DE 19640817 A1. Insbesondere ist eine Nukleinsäure bevorzugt, die von der *P. falciparum*-MSP-1-Sequenz abgeleitet ist und bei der ein Großteil der Plasmodium-Codons so ausgetauscht wurde, das die Codonhäufigkeit des synthetischen Gens der humanen entspricht, ohne dabei die Aminosäuresequenz zu verändem.

Gemäß einer bevorzugten Ausführungsform ist das MSP-1-Protein das MSP-1 des Isolats 3D7, das MSP-1-Proteins das nachfolgend als "MSP-1D" bezeichnet wird. Alternativ kann das MSP-1-Protein des FCB1-Stammes sein, das nachfolgend als "MSP-1 F" bezeichnet wird. Das MSP-1-Protein umfasst die angegebenen Fragmente dieser beiden Formen von MSP-1. Umfasst sind hierbei p42; p42 und p38; und p83, p30, p42 und p38. insbesondere ist die Kombination p83 und p30 sowie p38 und p42 bevorzugt. Die Lage der Fragmente ergibt sich hierbei aus Abbildung 1. Ferner umfasst werden auch das Fragment p42 von beiden MSP-1-Formen.

In einer bevorzugten Ausführungsform umfasst das im Vakzin enthaltene Virus einen zur Expression geeigneten Promotor, wobei die *msp*-1-kodierende Sequenz unter der Kontrolle des Promotors steht. Der Promotor kann hierbei ein MVA-Promotor sein, wobei der Promotor ein früher, intermediärer oder später Genpromotor oder eine Kombination davon sein kann. Jedoch sind auch nicht-MVA-Promotoren umfasst, die zur Expression in dem verwendeten Expressionssystem fähig sind. Hierbei können sowohl konstitutive als auch induzierbare Promotoren verwendet werden. Zur Large Scale Protein-Produktion kann hierbei ein starker Vaccinia Virus-Promotor, wie der synthetische späte oder frühe/späte Promotor oder das HybridVaccinia/T7 Polymeresasystem verwendet werden; zur induktion von MHC-Klasse I - restringierter zytotoktischer T-Zellantwort *in vivo* kann bevorzugt ein natürlich vorkommender früher oder Tandem-füher/später Promotor verwendet werden; weiterhin kann das *E. coli* lac repressor/Operatorsystem oder das HybridVaccinia/T7-System zur initiation der Genexpression verwendet werden; (Methods in Molecular Biology, Volume 62, Herausgeber Rocky S. Tuan, Humana Press, Broder und Earl, Seite 176, mit weiteren Nachweisen).

Gemäß einer weiteren bevorzugten Ausführungsform umfasst das Virus ferner einen Selektlonsmarker. Der Selektionsmarker ist hierbei zur Selektion und/oder zum Screening in bekannter Weise geeignet. Geeignete Selektionsmarker umfassen hierbei beispielsweise das *E. coli lacZ*-System, das Seiektions-System unter Verwendung von *E. coli* Xanthin-Guanin Phosphoribosyl Transferase (XGPRT)-Gen. Zusätzlich können Seiektionsmethoden verwendet werden, welche die Wirtszellspezifität der Viren verändern (Staib et al., 2000). Weitere im Stand der Technik bekannte Selektionsmarker können verwendet werden.

Gemäß einer weiteren bevorzugten Ausführungsform ist die Nukleinsäure am 5 Ende mit einer Signalpeptidsequenz kodierenden Nukleotidsequenz fusioniert. Wie aus dem Stand der Technik bekannt, werden die Signal- und Anker-Sequenzen aus *P. falciparum* bei Expression in Säugerzellen nicht erkannt, bzw. nicht korrekt prozessiert (Moran und Caras, 1994; Burghaus et al.,1999; Yang et al., 1997).

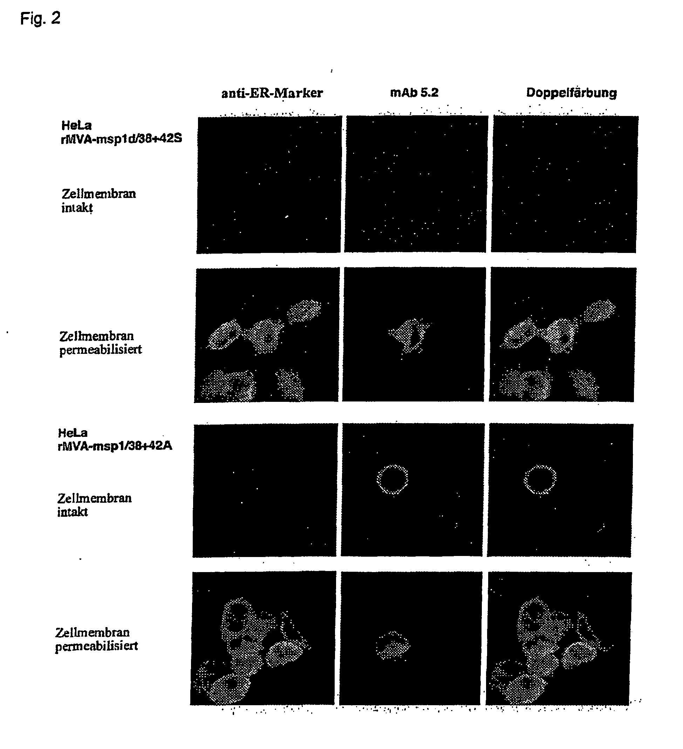

Das Problem der selektiven Steuerung des intrazellulären Gating wird durch die Verwendung der Signalsequenzen des humanen "Decay-Accelereting-Factors" (DAF) gelöst (Fig. 2). Geeignete Signalpeptidsequenzen sind spezifisch für höhere Eukaryonten. Beispiele für solche Signalsequenzen neben denen des Decay-Accelerating-Factors sind Immunglobuline oder Signalpeptide verschiedener Wachstumsfaktoren und Zytokine (von Heijne, 1985). Gemäß einer bevorzugten Ausführungsform steuert die Signalpeptidsequenz die Sekretion des Genproduktes, beispielsweise Cytokine, Antikörper etc..

Ferner sind Signalsequenzen bevorzugt, die zu einer GPI-Verankerung des C-Terminus des Genproduktes an der Zelloberfläche führen, wie bei den humanen DAF. Alternativ sind Peptidsequenzen bevorzugt, die die membranständige Lokalisierung des Genproduktes steuern, wie im Falle von Immunglobulinen des M-Isotyps oder des Vesicular Stomatitis Virus-G Proteins.

Gemäß einer weiteren bevorzugten Ausführungsform kann das Virus ferner geeignete Splice Donor- und Splice Akzeptor-Stellen enthalten, so dass eine entsprechend gespleißte mRNA entsteht, die zur Translation innerhalb des zu behandelnden Individuums geeignet ist. Die Nukleinsäure kann ferner eine Sequenz enthalten, die als Ribosomenbindungsstelle geeignet ist.

Als pharmakologisch verträgliche Träger sind hierbei sämtliche im Stand der Technik bekannten Träger und Verdünnungsmittel geeignet. Sofern eine bestimmte Applikationsart beabsichtigt ist, kann der pharmakologisch verträgliche Träger in bekannter Weise ausgewählt oder geändert werden.

Das Vakzin kann subkutan, intramuskulär, intravenös, transdermal, intraperitoneal oder oral verabreicht werden. Das Vakzin wird zur Prophylaxe und/oder Therapie von Malaria bei Mensch und Tier angegeben.

Gemäß einer bevorzugten Ausführungsform kann das Vakzin ferner MSP-1, und / oder ein im Anspruch 1 aufgeführtes Fragment bzw. eine im Anspruch 1 aufgeführte Fragmentkombination davon enthalten.

Besonders bevorzugt ist das MSP-1-Protein hierbei rekombinant hergesteilt, insbesondere rekombinant in *E. coli.* Die MSP-1 kodierende Nukleinsäure ist, vorzugsweise eine solche, die bezüglich ihres AT-Gehaltes verringert ist Insbesondere bevorzugt ist eine solche Nukleinsäure wie in DE-19640817 A1 beschrieben, bei der Insbesondere *Plasmodium* falciparum-Codons gegen humane Codons ausgetauscht sind, ohne die Aminosäure-Sequenz hierbei zu verändem.

Sofern das Vakzin sowohl das rekombinante Virus als auch MSP-1, oder ein Fragment davon umfasst, so kann das Vakzin In Kit-Form vorlieben. Es Ist somit geeignet zur simultanen, sequenziellen oder getrennten Verabreichung der beiden Komponenten des Vakzins.

Die nachfolgenden Beispiele sollen die Erfindung veranschaulichen.

### Fig. 1: Primäre Struktur der MSP-1 aus den P. fa/ciparum Stämmen FCB-1 und MAD20.

Die Pfeile oberhalb der Sequenz kennzeichnen die Prozessierungsstellen der nativen Proteine (Holder et al., 1987), welche MSP-1 in die Fragmenten p83, p30, p38 und p42 teilen, die als Komplex auf der Parasitenoberfläche verankert sind. Im zweiten Prozessierungsschritt wird p42 zu p33 und p19 gespalten. Die Pfeile unterhalb der Darstellungen bezeichnen die einmalig vorkommenden Endonuklease-Schnittstellen der synthetischen DNA-Sequenzen. Abkürzungen: SP = Signalpeptid, GA = GPI-Anker

### Fig. 2: Expression von msp1d-38/42 in mit rekombinanten MVA-infizierten HeLa-Zellen

HeLa-Zellen wurden mit rMVA-msp1d/38+42S bzw. rMVA-msp1d/38+42A infiziert und anschließend fixiert. Die zweite und vierte Zelle zeigt jeweils Zellen, die nach der Fixierung mit Triton X-100 permeabilisiert wurden, während die Membran der Zellen in Zeile eins und drei intakt blieb. Die so behandelten Zellen wurden mit mAk 5.2, der ein für MSP-1spezifisches, konformationelles Epitop in C-terminalen Teil des MSP-1-Fragments p19 erkennt und einem polyklonalen Serum, das das ER-Protein Sec61beta erkennt (anti-ER-Marker), als erstem Antikörper inkubiert, diese über Cy3-konjugierte anti-Maus-IgG (erkennt mAk 5.2) bzw. FITC-konjugierte anti-Kaninchen-IgG (erkennt anti-Sec61beta) farblich markiert und anschließend im konfokalen Mikroskop analysiert. Wurden die Zellen mit rMVA-msp1d/38+42S bzw. rMVA-msp1d/38+42A infiziert und permeabiliziert, so kann das Signal für MSP-1D-38/42 (mAk 5.2) kolokalisiert werden mit dem ER-Marker. Blieben die Zellen hingegen unversehrt, so wird MSP-1 D-38/42 nur im Fall der Infektion mit rMVA-msp1d/38+42A auf der Oberfläche der infizierten Zellen erkannt. Der ER-Marker fungierte hier als Kontrolle für die Unversehrtheit der Zellmembran.
Abkürzungen: ER = Endoplasmatisches Retikulum; mAk = monoklonaler Antikörper

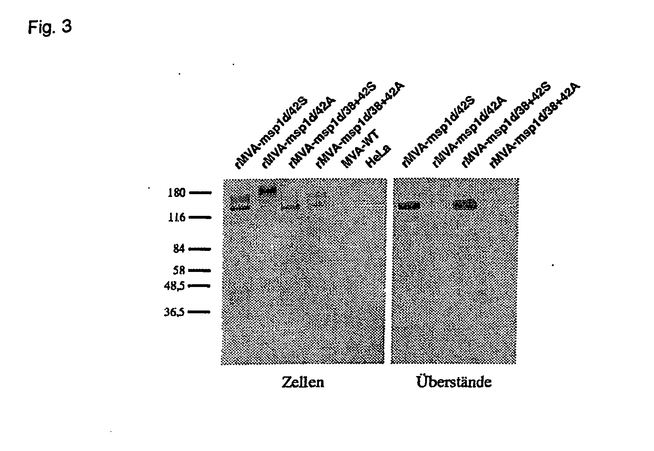

### Fig. 3: Nachweis von MSP-1D-42 und MSP-1D-38/42 in mit rekombinanten MVA infizierten HeLa-Zellen mittels Immunoblot

**A** HeLa-Zellen wurden mit rMVA-msp1d/42S, rMVA-msp1d/42A, rMVA-msp1d/38+42S bzw. rMVA-msp1d/38+42A infiziert und über Nacht inkubiert. Proben des Überstands und der zellulären Fraktion wurden über SDS-Gel-Elektrophorese unter nicht reduzierenden Bedingungen aufgetrennt, auf eine PVDF-Membran transferiert und mittels mAb 5.2 als Erstantikörper nachgewiesen. Nur Chimäre aus DAF-Signalsequenz und den entsprechenden MSP-1D-Fragmenten lassen sich in den Überständen der infizierten Zellen nachweisen, während die intrazelluläre Expression in allen mit rekombinanten MVA infizierten Zellen ein Signal liefert.

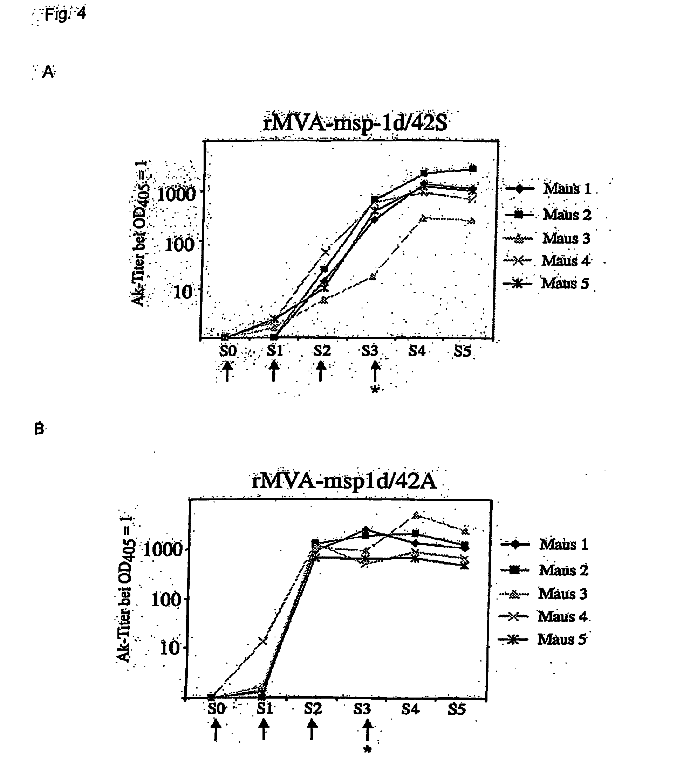

### Fig. 4: Entwicklung der humoralen Immunantwort nach drei Immunisierungen mit rMVA-msp1d/42S bzw. rMVA-msp1d/42A und einer Immunisierung mit MSP-1D-HX42 aus E. coli

In **A** ist die Analyse der humoralen Immnunantwort mittels ELISA mit rekombinant hergestelltem MSP-1D-HX42 aufgereinigt aus *E. coli* als Antigen dargestellt. Die Kurven zeigen die p42 spezifische Antikörperentwicklung, gemessen an der OD₄₀₅ = 1. Die Mäuse wurden jeweils im Abstand von drei Wochen mit 10⁶ IU (Erstimmunisierung, zeitgleich mit der Blutentnahme S0) bzw. 10⁸ IU (1. und 2. Boost, zeitgleich mit S1 und S2) rMVA-msp1d/42S immunisiert. Zusätzlich wurde den Mäusen nach weiteren vier Wochen je 5µg MSP-1 D-HX42 aus *E. coli* in unkompletten Freund'schem Adjuvans subcutan injiziert (eine Woche nach der Blutentnahme S3). S0 bis S5 stellen die Zeitpunkte der Blutentnahme dar, dabei wurde das Blut S0 bis S3 jeweils im Abstand von drei Wochen entnommen, die Entnahme von S4 und S5 erfolgte jeweils im Abstand von 4 Wochen. die Pfeile markieren die Zeitpunkte der Immunisierungen. Der Stern markiert die vierte Immunisierung mit MSP-1 D-HX42 aus *E. coli.*
**B** zeigt die gleiche Analyse für die Immunisierung mit rMVA-msp1 d/42A.

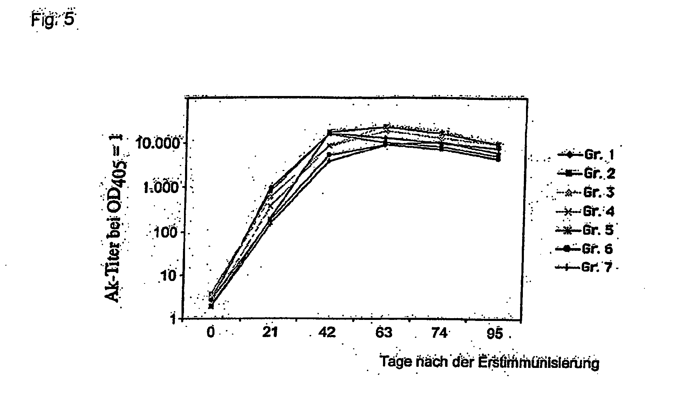

### Fig. 5: Entwicklung der humoralen Immunantwort nach Immunisierungen mit rMVA-msp1d/S bzw. rMVA-msp1d/83+30/38+42A in Kombination mit Immunisierung mit MSP-1D aus E. coli

Die humorale Immunantwort wurde mittels ELISA unter Verwendung von rekombinant hergestelltem MSP-1 D aufgereinigt aus *E. coli* als Antigen ermittelt. Wie in Fig.4 zeigen die Kurven die MSP-1 D-spezifische Antikörperentwicklung, gemessen an der OD₄₀₅ = 1. Die Mäuse wurden jeweils im Abstand von 3 Wochen immunisiert (in der Abbildung durch Pfeile gekennzeichnet). Die den Gruppen zugeordneten Immunisierungsstrategien setzten sich wie folgt zusammen:
Gr. 1: 20 µg MSP-1 D (Tag 0), 10⁸ IU rMVA-msp1d/S (Tag 21), 5 Mäuse
Gr. 2: 20 µg MSP-1 D (Tag 0), 10⁸ IU rMVA-msp1d/A (Tag 21), 5 Mäuse
Gr. 3: 20 µg MSP-1 D (Tag 0), 10⁸ IU rMVA-msp1d/S (Tag 21), 20 µg MSP-1 D (Tag 42), 10 Mäuse
Gr. 4: 20 µg MSP-1 D (Tag 0), 10⁸ IU rMVA-msp1d/S (Tag 21), 10⁸ IU rMVA-msp1d/S (Tag 42), 10 Mäuse
Gr. 5: 20 µg MSP-1 D (Tag 0), 10⁸ IU rMVA-msp1d/83+30/38+42A (Tag 21), 20 µg MSP-1 D (Tag 42) 9 Mäuse
Gr. 6: 20 µg MSP-1D (Tag 0), 10⁸ IU rMVA-msp1d/83+30/38+42A (Tag 21), 10⁸ IU rMVA-msp1d183+30/38+42A (Tag 42), 10 Mäuse
Gr. 7: 20 µg MSP-1 D (Tag 0), 10⁸ IU rMVA-msp1d/A (Tag 21), 20 µg MSP-1 D (Tag 42), 5 Mäuse

Im folgenden werden rekombinante Viren, welche zur Herstellung von MSP-1 in seiner Oberflächen-verankerten Form führen, mit "A" und solche zur Sekretion von MSP-1 durch "S" gekennzeichnet.

**Tabelle 1 Vollständige Liste der im Rahmen der Erfindung hergestellten Virus-Konstrukte:**

| | rMVA-msp1d | rMVA-msp1f |
|---|---|---|
| sekretiertes MSP-1 | rMVA-msp1d/S | |
| | rMVA-msp1d/83S | |
| | rMVA-msp1d/83+30S | |
| | rMVA-msp1d/42S | |
| | rMVA-msp1d/38+42S | |
| Oberflächen-verankertes MSP-1 | rMVA-msp1d/A | rMVA-msp1f/A |
| | rMVA-msp1d/83A | rMVA-msp1f/83+30/38+42A |
| | rMVA-msp1d/83+30A | rMVA-msp1f/38+42A |
| | rMVA-msp1d/42A | |
| | rMVA-msp1d/38+42A | |
| | rMVA-msp1d/83+30/38+42A | |

Die Herstellung von MSP-1 bzw. der Fragmente und die Lokalisation der Proteine in der infizierten Zelle wurde mittels konfokaler Mikroskopie belegt und ist exemplarisch für die Infektion von HeLa-Zellen durch rMVA-*msp-*1d/38+42S und rMVA-msp1d/38+42A dargestellt (Fig. 2).
Die Sekretion aller MSP-1-Varianten aus mit rekombinanten MVA infizierten Zellen wurde über Immunoblot-Analysen zellulärer Überstände nachgewiesen und ist hier exemplarische für rMVA-msp1d/42S und rMVA-msp1d/38+42S dargestellt (Fig. 3).

Anschließend wurden die rekombinanten MVA in Immunisierungsversuchen an Mäusen auf ihre immunogene Wirkung bezüglich der humoralen Immunantwort untersucht. Dabei induzierten für *msp-1* rekombinanten MVA hohe Antikörpertiter gegen das Parasitenprotein, die über ELISA bestimmt wurden. Die p42-spezfischen Antikörpertiter und das beobachtete, unterschiedliche Immunisierungspotential der durch rekombinante MVA hergestellten Oberflächen-verankerten bzw. sekretierten Proteine wird exemplarisch für Immunisierungen mit rMVA-msp1d/42S und rMVA-msp1d/42A dargestellt (Fig. 4).

### Literatur

Aidoo, M., Lalvani, A., Whittle, H. C., Hill, A. V., and Robson, K. J. (1997). Recombinant vaccinia viruses for the characterization of Plasmodium falciparum-specific cytotoxic T lymphocytes: recognition of processed antigen despite limited re-stimulation efficacy. Int Immunol 9, 731-7.
Allsopp, C. E., Plebanski, M., Gilbert, S., Sinden, R. E., Harris, S., Frankel, G., Dougan, G., Hioe, C., Nixon, D., Paoletti, E., Layton, G., and Hill, A. V. (1996). Comparison of numerous delivery systems for the induction of cytotoxic T lymphocytes by immunization. Eur J Immunol 26, 1951-9.
Antoine, G., Scheiflinger, F., Domer, F, and Falkner, F. G. (1998). The complete genomic sequence of the modified vaccinia Ankara strain: Comparison with other Orthopoxviruses. Virology 244, 365-396.
Chang, S. P., Case, S. E., Gosnell, W. L., Hashimoto, A., Kramer, K. J., Tam, L. Q., Hashiro, C. Q., Nikaido, C. M., Gibson, H. L., Lee-Ng, C. T., Barr, P. J., Yokota, B. T., and Hut, G. S. (1996). A recombinant baculovirus 42-kilodalton C-terminal fragment of Plasmodium falciparum merozoite surface protein 1 protects Aotus monkeys against malaria. Infect Immun 64, 253-61.
Daly, T. M., and Long, C. A. (1993). A recombinant 15-kilodalton carboxyl-terminal fragment of Plasmodium yoelii yoelii 17XL merozoite surface protein 1 induces a protective immune response in mice. Infect. Immun. 61, 2462-7.
Degano, P., Schneider, J., Hannan, C. M., Gilbert, S. C., and Hill, A. V. (1999). Gene gun intradermal DNA immunization followed by boosting with modified Vaccinia virus Ankara: enhanced CD8+ T cell immunogenicity and protective efficacy in the influenza and malaria models. Vaccine 18, 623-32.
Egan, A. F., Blackman, M. J., and Kaslow, D. C. (2000). Vaccine efficacy of recombinant Plasmodium falciparum merozoite surface protein 1 in malaria-naive, -exposed, and/or - rechallenged Aotus vociferans monkeys. Infect Immun 68, 1418-27.
Etlinger, H. M., and Altenburger, W. (1991). Overcoming inhibition of antibody responses to a malaria recombinant vaccinia virus caused by prior exposure to wild type virus. Vaccine 9, 470-2.
Genton, B., Al-Yaman, F., Anders, R., Saul, A., Brown, G., Pye, D., Irving, D. O., Briggs, W. R., Mai, A., Ginny, M., Adiguma, T., Rare, L, Giddy, A., Reber-Liske, R., Stuerchler, D., and Alpers, M. P. (2000). Safety and immunogenicity of a three-component blood-stage malaria vaccine in adults living in an endemic area of Papua New Guinea. Vaccine 18, 2504-11.
Gilbert, S. C., Schneider, J., Plebanski, M., Hannan, C. M., Blanchard, T. J., Smith, G. L, and Hill, A. V. (1999). Ty virus-like particles, DNA vaccines and Modified Vaccinia Virus Ankara; comparisons and combinations. Biol Chem 380, 299-303.
Hirunpetcharat, C., Tian, J. H., Kaslow, D. C., van Rooijen, N., Kumar, S., Berzofsky, J. A., Miller, L. H., and Good, M. F. (1997). Complete protective immunity induced in mice by immunization with the 19-kilodalton carboxyl-terminal fragment of the merozoite surface protein-1 (MSP1[19]) of Plasmodium yoelii expressed in Saccharomyces cerevisiae: correlation of protection with antigen-specific antibody titer, but not with effector CD4+ T cells. J Immunol 159, 3400-11.
Holder, A. A., and Freeman, R. R. (1981). Immunization against blood-stage rodent malaria using purified parasite antigens. Nature. 294, 361-364.
Holder, A. A., Sandhu, J. S., Hillman, Y., Davey, L. S., Nicholls, S. C., Cooper, H., and Lockyer, M. J. (1987). Processing of the precursor to the major merozoite surface antigens of Plasmodium falciparum. Parasitology 94, 199-208.
Kaslow, D. C., Isaacs, S. N., Quakyi, I. A., Gwadz, R. W., Moss, B., and Keister, D. B. (1991). Induction of Plasmodium falciparum transmission-blocking antibodies by recombinant vaccinia virus. Science 252, 1310-1313.
Keitel, W. A., Kester, K. E., Atmar, R. L., White, A. C., Bond, N. H., Holland, C. A., Krzych, U., Palmer, D. R., Egan, A., Diggs, C., Ballou, W. R., Hall, B. F., and Kaslow, D. (1999). Phase I trial of two recombinant vaccines containing the 19kd carboxy terminal fragment of Plasmodium falciparum merozoite surface protein 1 (msp-1(19)) and T helper epitopes of tetanus toxoid. Vaccine 18, 531-9.
Kumar, S., Yadava, A., Keister, D. B., Tian, J. H., Ohl, M., Perdue-Greenfield, K. A., Miller, L. H., and Kaslow, D. C. (1995). Immunogenicity and in vivo efficacy of recombinant Plasmodium falciparum merozoite surface protein-1 in Aotus monkeys. Mol Med 1, 325-32.
Ling, I. T., Ogun, S. A., and Holder, A. A. (1994). Immunization against malaria with a recombinant protein. Parasite Immunol 16, 63-7.
Majarian, W. R., Daly, T. M., Weidanz, W. P., and Long, C. A. (1984). Passive immunization against murine malaria with an IgG3 monoclonal antibody. J. Immunol. 132, 3131-3137.
Matsumoto, S., Yukitake, H., Kanbara, H., and Yamada, T. (1999). Long-lasting protective immunity against rodent malaria parasite infection at the blood stage by recombinant BCG secreting merozoite surface protein-1. Vaccine 18, 832-4.
Meyer, H., Sutter, G., and Mayr, A. (1991). Mapping of deletions in the genome of the highly attenuated vaccinia virus MVA and their influence on virulence. J Gen Virol 72, 1031-8.
Moran, P., and Caras, I. W. (1994). Requirements for glycosylphosphatidylinositol attachment are similar but not identical in mammalian cells and parasitic protozoa. J Cell Biol 125, 333-43.
Moss, B., Carroll, M. W., Wyatt, L. S., Bennink, J. R., Hirsch, V. M., Goldstein, S., Elkins, W. R., Fuerst, T. R., Lifson, J. D., Piatak, M., Restifo, N. P., Overwijk, W., Chamberlain, R., Rosenberg, S. A., and Sutter, G. (1996). Host range restricted, non-replicating vaccinia virus vectors as vaccine candidates. Adv Exp Med Biol 397, 7-13.
Ockenhouse, C. F., Sun, P. F., Lanar, D. E., Wellde, B. T., Hall, B. T., Kester, K., Stoute, J. A., Magill, A., Krzych, U., Farley, L, Wirtz, R. A., Sadoff, J. C., Kaslow, D. C., Kumar, S., Church, L W., Crutcher, J. M., Wizel, B., Hoffman, S., Lalvani, A., Hill, A. V., Tine, J. A., Guito, K. P., de Taisne, C., Anders, R., Ballou, W. R., and et al. (1998). Phase I/IIa safety, immunogenicity, and efficacy trial of NYVAC-Pf7, a pox-vectored, multiantigen, multistage vaccine candidate for Plasmodium falciparum malaria. J Infect Dis 177, 1664-73.
Perrin, L. H., Merkli, B., Loche, M., Chizzolini, C., Smart, J., and Richle, R. (1984). Antimalarial immunity in Saimiri monkeys. Immunization with surface components of asexual blood stages. J. Exp. Med. 160, 441-451.
Plebanski, M., Gilbert, S. C., Schneider, J., Hannan, C. M., Layton, G., Blanchard, T., Becker, M., Smith, G., Butcher, G., Sinden, R. E., and Hill, A. V. (1998). Protection from Plasmodium berghei infection by priming and boosting T cells to a single class I-restricted epitope with recombinant carriers suitable for human use. Eur J Immunol 28, 4345-55.
Pye, D., Edwards, S. J., Anders, R. F., O'Brien, C. M., Franchina, P., Corcoran, L. N., Monger, C., Peterson, M. G., Vandenberg, K. L., Smythe, J. A., Westley, S. R., Coppel, R. L., Webster, T. L., Kemp, D. J., Hampson, A. W., and Langford, C. J. (1991). Failure of recombinant vaccinia viruses expressing Plasmodium falciparum antigens to protect Saimiri monkeys against malaria. Infect. Immun. 59, 2403-2411.
Riley, E. M., Allen, S. J., Wheeler, J. G., Blackman, M. J., Bennett, S., Takacs, B., Schonfeld, H. J., Holder, A. A., and Greenwood, B. M. (1992). Naturally acquired cellular and humoral immune responses to the major merozoite surface antigen (PfMSP1) of Plasmodium falciparum are associated with reduced malaria morbidity. Parasite Immunol. 14, 321-337.
Riley, E. M., Morris-Jones, S., Blackman, M. J., Greenwood, B. M., and Holder, A. A. (1993). A longitudinal study of naturally acquired cellular and humoral immune responses to a merozoite surface protein (MSP1) of Plasmodium falciparum in an area of seasonal malaria transmission. Parasite Immunol. 15, 513-524.
Saul, A., Lawrence, G., Smillie, A., Rzepczyk, C. M., Reed, C., Taylor, D., Anderson, K., Stowers, A., Kemp, R., Allworth, A., Anders, R. F., Brown, G. V., Pye, D., Schoofs, P., Irving, D. O., Dyer, S. L., Woodrow, G. C., Briggs, W. R., Reber, R., and Sturchler, D. (1999). Human phase I vaccine trials of 3 recombinant asexual stage malaria antigens with Montanide ISA720 adjuvant. Vaccine 17, 3145-59.
Schneider, J., Gilbert, S. C., Blanchard, T. J., Hanke, T., Robson, K. J., Hannan, C. M., Becker, M., Sinden, R., Smith, G. L., and Hill, A. V. (1998). Enhanced immunogenicity for CD8+ T cell induction and complete protective efficacy of malaria DNA vaccination by boosting with modified vaccinia virus Ankara. Nat Med 4, 397-402.
Siddiqui, W. A., Tam, L. Q., Kramer, K. J., Hui, G. S., Case, S. E., Yamaga, K. M., Chang, S. P., Chan, E. B., and Kan, S. C. (1987). Merozoite surface coat precursor protein completely protects Aotus monkeys against Plasmodium falciparum malaria. Proc. Natl. Acad. Sci. USA 84, 3014-3018.
Staib, C., Drexler, 1., Ohlmann, M., Wintersperger, S., Erfle, V., and Sutter, G. (2000). Transient host range selection for genetic engineering of modified vaccinia virus Ankara. Biotechniques 28, 1137-42, 1144-6, 1148.
Stickl, H., Hochstein-Mintzel, V., Mayr, A., Huber, H. C., Schafer, H., and Holzner, A. (1974). [MVA vaccination against smallpox: clinical tests with an attenuated live vaccinia virus strain (MVA) (author's transl)]. Dtsch Med Wochenschr 99, 238-92.
Stowers, A. W., Cioce, V., Shimp, R. L., Lawson, M., Hui, G., Muratova, O., Kaslow, D. C., Robinson, R., Long, C. A., and Miller, L. H. (2001). Efficacy of two altemate vaccines based on Plasmodium falciparum merozoite surface protein 1 in an Aotus challenge trial. Infect Immun 69, 1536,46.
Sutter, G., and Moss, B. (1992). Nonreplicating vaccinia vector efficiently expresses recombinant genes. Proc Natl Acad Sci U S A 89, 10847-51.
Tanabe, K., Mackay, M., Goman, M., and Scaife, J. G. (1987). Allelic dimorphism in a surface antigen gene of the malaria parasite Plasmodium falciparum. J. Mol. Biol. 195, 273-287.
Tartaglia, J., Perkus, M. E., Taylor, J., Norton, E. K., Audonnet, J. C., Cox, W. I., Davis, S. W., van der Hoeven, J., Meignier, B., Riviere, M., and et al. (1992). NYVAC: a highly attenuated strain of vaccinia virus. Virology 188, 217-32.
Tian, J. H., Miller, L. H., Kaslow, D. C., Ahlers, J., Good, M. F., Alling, D. W., Berzofsky, J. A., and Kumar, S. (1996). Genetic regulation of protective immune response in congenic strains of mice vaccinated with a subunit malaria vaccine. J Immunol 157, 1176-83.
Tine, J. A., Lanar, D. E., Smith, D. M., Wellde, B. T., Schultheiss, P., Ware, L. A., Kauffman, E. B., Wirtz, R. A., De Taisne, C., Hui, G. S., Chang, S. P., Church, P., Hollingdale, M. R., Kaslow, D. C., Hoffman, S., Guito, K. P., Ballou, W. R., Sadoff, J. C., and Paoletti, E. (1996). NYVAC-Pf7: a poxvirus-vectored, multiantigen, multistage vaccine candidate for Plasmodium falciparum malaria. Infect Immun 64, 3833-44.
Tolle, R., Fruh, K., Doumbo, O., Koita, O., N'Diaye, M., Fischer, A., Dietz, K., and Bujard, H. (1993). A prospective study of the association between the human humoral immune response to Plasmodium falciparum blood stage antigen gp190 and control of malarial infections. Infect Immun 61, 40-7.
von Heijne, G. (1985). Signal sequences. The limits of variation. J Mol Biol 184, 99-105.
Wunderlich, G., Moura, I. C., and del Portillo, H. A. (2000). Genetic immunization of BALB/c mice with a plasmid bearing the gene coding for a hybrid merozoite surface protein 1-hepatitis B virus surface protein fusion protects mice against lethal Plasmodium chabaudi chabaudi PC1 infection. Infect Immun 68, 5839-45.
Yang, S., Carroll, M. W., Torres-Duarte, A. P., Moss, B., and Davidson, E. A. (1997). Addition of the MSA1 signal and anchor sequences to the malaria merozoite surface antigen 1 C-terminal region enhances immunogenicity when expressed by recombinant vaccinia virus. Vaccine 15, 1303-13.

## Patentansprüche

1. Vakzin umfassend
a) ein rekombinantes MVA-Virus, umfassend eine ein Fragment oder Fragmente von *Plasmodium falciparum* MSP-1-Protein kodierende Nukleinsäure; und
b) einen pharmakologischen verträglichen Träger,
wobei das durch die Nukleinsäure kodierte MSP-1 Fragment bzw. die MSP-1 Fragmente ausschließlich ausgewählt ist/sind aus:
• p42;
• p42 und p38;
• p83, p30, p42 und p38.

2. Vakzin nach Anspruch 1, **dadurch gekennzeichnet, dass** das MSP-1-Protein das MSP-1-Protein des Isolates 3D7 oder das MSP-1-Protein des FCB1-Stammes ist.

3. Vakzin nach einem der Ansprüche 1 oder 2, **dadurch gekennzeichnet, dass** die MSP-1 kodierende Nukleinsäure gegenüber der Wildtyp-Sequenz in ihrem AT-Gehalt verringert ist.

4. Vakzin nach einem der Ansprüche 1 bis 3, **dadurch gekennzeichnet, dass** die Nukleinsäure unter der Kontrolle eines Promotors steht.

5. Vakzin nach einem der Ansprüche 1 bis 4, **dadurch gekennzeichnet, dass** die Nukleinsäure am 5' Ende mit einer eine Signalpeptidsequenz kodierenden Nukleotidsequenz fusioniert ist.

6. Vakzin nach Anspruch 5, **dadurch gekennzeichnet, dass** die Signalpeptidsequenz die Sekretion des Genproduktes steuert.

7. Vakzin nach Anspruch 5, **dadurch gekennzeichnet, dass** die Signalpeptidsequenz die membranständige Lokalisierung des Genproduktes steuert.

8. Vakzin nach Anspruch 5, **dadurch gekennzeichnet, dass** die Signalsequenz die GPI-Verankerung des Genproduktes steuert.

9. Vakzin gemäss Anspruch 8, **dadurch gekennzeichnet, dass** das Vakzin ferner als Bestandteil c) MSP-1 und/oder ein im Anspruch 1 aufgeführtes Fragment bzw. eine im Anspruch 1 aufgeführte Fragmentkombination davon enthält.

10. Vakzin gemäss Anspruch 9, **dadurch gekennzeichnet, dass** die Bestandteile a) und c) simultan, sequenziell oder getrennt verabreicht werden können.

11. Verwendung des Vakzins gemäss einem der Ansprüche 1 bis 7 zur Herstellung eines Medikaments zur Prophylaxe und/oder Therapie von Malaria.

12. Verwendung des Vakzins gemäss einem der Ansprüche 1 bis 6 und von MSP-1 und/oder einem im Anspruch 1 aufgeführten Fragment oder einer im Anspruch 1 aufgeführten Fragmentkombination davon zur Herstellung eines Medikaments zur Prophylaxe und/oder Therapie von Malaria

## Claims

1. Vaccine comprising
a) a recombinant MVA virus comprising a nucleic acid coding for a fragment or fragments of *Plasmodium falciparum* MSP-1 protein; and
b) a pharmacologically acceptable carrier,
wherein the MSP-1 fragment or the MSP-1 fragments coded for by the nucleic acid is/are exclusively selected from:
• p42;
• p42 and p38;
• p83, p30, p42 and p38.

2. Vaccine according to claim 1, **characterised in that** the MSP-1 protein is the MSP-1 protein of the isolate 3D7 or the MSP-1 protein of the FCB1 strain.

3. Vaccine according to any one of the claims 1 or 2, **characterised in that** the AT content of the nucleic acid coding for MSP-1 is reduced in comparison to the wild type sequence.

4. Vaccine according to any one of the claims 1 to 3, **characterised in that** the nucleic acid is under the control of a promoter.

5. Vaccine according to any one of the claims 1 to 4, **characterised in that** the nucleic acid is fused at the 5' end to a nucleotide sequence coding for a signal peptide sequence.

6. Vaccine according to claim 5, **characterised in that** the signal peptide sequence controls the secretion of the gene product.

7. Vaccine according to claim 5, **characterised in that** the signal peptide sequence controls the membrane-bound localisation of the gene product.

8. Vaccine according to claim 5, **characterised in that** the signal sequence controls the GPI anchoring of the gene product.

9. Vaccine according to claim 8, **characterised in that** the vaccine further comprises, as component c), MSP-1 and/or a fragment thereof specified in claim 1 or a fragment combination thereof specified in claim 1.

10. Vaccine according to claim 9, **characterised in that** components a) and c) can be administered simultaneously, sequentially or separately.

11. Use of the vaccine according to any one of the claims 1 to 7 for the preparation of a medicament for the prophylaxis and/or therapy of malaria.

12. Use of the vaccine according to any one of the claims 1 to 6 and of MSP-1 and/or a fragment thereof specified in claim 1 or a fragment combination thereof specified in claim 1 for the preparation of a medicament for the prophylaxis and/or therapy of malaria.

## Revendications

1. Vaccin comprenant
a) un virus MVA recombinant, comprenant un acide nucléique codant un fragment ou des fragments de la protéine MSP-1 de *Plasmodium falciparum ;* et
b) un véhicule pharmacologiquement acceptable,
dans lequel le fragment de MSP-1 ou les fragments de MSP-1 codés par l'acide nucléique est/sont choisi(s) exclusivement parmi :
• p42
• p42 et p38 ;
• p83, p30, p42 et p38.

2. Vaccin selon la revendication 1, **caractérisé en ce que** la protéine MSP-1 est la protéine MSP-1 de l'isolat 3D7 ou la protéine MSP-1 de la souche FCB1.

3. Vaccin selon l'une des revendications 1 ou 2, **caractérisé en ce que** l'acide nucléique codant la MSP-1 a une teneur réduite en A-T par rapport à la séquence de type sauvage.

4. Vaccin selon l'une des revendications 1 à 3, **caractérisé en ce que** l'acide nucléique est sous le contrôle d'un promoteur.

5. Vaccin selon l'une des revendications 1 à 4, **caractérisé en ce que** l'acide nucléique est fusionné à l'extrémité 5' avec une séquence nucléotidique codant une séquence de peptide signal.

6. Vaccin selon la revendication 5, **caractérisé en ce que** la séquence de peptide signal commande la sécrétion du produit génique.

7. Vaccin selon la revendication 5, **caractérisé en ce que** la séquence de peptide signal commande la localisation membranaire du produit génique.

8. Vaccin selon la revendication 5, **caractérisé en ce que** la séquence signal commande l'ancrage GPI du produit génique.

9. Vaccin selon la revendication 8, **caractérisé en ce que** le vaccin contient en outre, comme composant c), la MSP-1 et/ou un fragment de celle-ci cité dans la revendication 1 ou une combinaison de fragments de celle-ci cités dans la revendication 1.

10. Vaccin selon la revendication 9, **caractérisé en ce que** les composants a) et c) peuvent être administrés simultanément, séquentiellement ou séparément.

11. Utilisation du vaccin selon l'une des revendications 1 à 7 pour la préparation d'un médicament pour la prophylaxie et/ou la thérapie du paludisme.

12. Utilisation du vaccin selon l'une des revendications 1 à 6 et de la MSP-1 et/ou d'un fragment de celle-ci cité dans la revendication 1 ou d'une combinaison de fragments de celle-ci cités dans la revendication 1 pour la préparation d'un médicament pour la prophylaxie et/ou la thérapie du paludisme.
